(19) Europäisches Patentamt
European Patent Office
Office européen des brevets

(11) Veröffentlichungsnummer: **0 414 020 B1**

# (12) EUROPÄISCHE PATENTSCHRIFT

(45) Veröffentlichungstag der Patentschrift: **21.09.94**

(51) Int. Cl.[5]: **C07D 207/273**, C07D 303/48, C07D 301/14

(21) Anmeldenummer: **90115023.5**

(22) Anmeldetag: **04.08.90**

## (54) Verfahren zur Herstellung von Clausenamid und Neoclausenamid und deren Derivate.

(30) Priorität: **19.08.89 DE 3927370**

(43) Veröffentlichungstag der Anmeldung:
**27.02.91 Patentblatt 91/09**

(45) Bekanntmachung des Hinweises auf die Patenterteilung:
**21.09.94 Patentblatt 94/38**

(84) Benannte Vertragsstaaten:
**AT BE CH DE DK ES FR GB GR IT LI LU NL SE**

(56) Entgegenhaltungen:
**EP-A- 0 222 163**
**EP-A- 0 244 717**
**EP-A- 0 362 556**
**DE-A- 2 811 256**
**DE-A- 3 431 257**
**DE-A- 3 537 075**
**DE-A- 3 616 989**

**JOURNAL OF ORGANIC CHEMISTRY, vol. 52, no. 19, 18 September 1987, Seiten 4352 -4358; W. HARTWIG et al.: "Diastereoselective and Enantioselective Total Synthesis of the Hepatoprotective agent Clausenamide"**

(73) Patentinhaber: **BAYER AG**
**D-51368 Leverkusen (DE)**

Patentinhaber: **CHINESE ACADEMY OF MEDICAL SCIENCES**
**1 Xian Nong Tan Street**
**Beijing 100050 (CN)**

(72) Erfinder: **Huang, Liang, Prof., Institute of materia Medica**
**Chinese Academy of Medical Sciences,**
**1 Xian Nong**
**Tan Street, Beijing 100050 (CN)**
Erfinder: **Liu, Geng-tao, Prof., Institute of materia Medica**
**Chinese Academy of Medical Sciences,**
**1 Xian Nong**
**Tan Street, Beijing 100050 (CN)**

(74) Vertreter: **Dänner, Klaus, Dr.**
**Bayer AG**
**Konzernverwaltung RP**
**Patente Konzern**
**D-51368 Leverkusen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, vol. 106, no. 19, 11 Mai 1987 Columbus, Ohio, USA M. YANG et al.:"Isolation and structural elucidation of clausenamide from the leaves of Clausena lansium (Lour.) Skeels" Seite 401; ref. no. 153109Z & Yaoxue Xuebao 1987, 22(1), 33-40

JOURNAL OF THE AMERICAN CHEMICAL SOCIETY, vol. 107, 1985, Seiten 1438 - 1439; S. HANESSIAN et al.: "A New Synthetic Strategy for the Penems. Total Synthesis of (5 R, 6S, 8R)-6-( -Hydroxyethyl) - 2 - (hydroxymethyl) penem-3-carboxylic Acid"

## Beschreibung

Die Erfindung betrifft ein Verfahren zur Herstellung von (±) 3(S*),4(R*), 5(R*), 7(S*)-1-methyl-3-hydroxy-4-phenyl-5-(α-hydroxybenzyl)pyrrolidin-2-on (Clausenamid) und (±) 3(S*), 4(R*), 5(S*), 7(R*)-1-methyl-3-hydroxy-4-phenyl-5-(α-hydroxybenzyl)pyrrolidin-2-on (Neoclausenamid) sowie deren Derivate.

Clausenamid, Neoclausenamid, einige ihrer Derivate und Verfahren zu ihrer Herstellung sind bereits bekannt [vgl. DE-A1 36 16 989; J. Org. Chem. 52 (19), 4352 - 4358; Phytochemistry 28 (2), 445 - 450; DE-A1 36 32 589, DE-A1 35 37 075 und DE-A1 34 31 257]. Außerdem ist bekannt, daß Rutaceae Clausena anicata in bestimmten Teilen Afrikas als Volksmedizin verwendet wird [vgl. J. Mester et al., Planta Medica 32, 81 (1977)]. Es ist ebenfalls bekannt, daß der rohe Extrakt von Clausena indica Oliv. cardiovaskuläre Aktivität besitzt und daß zwei aus Clausena pentaphylla (Roxb.) dünnschichtchromatographisch isolierte Cumarinderivate, Clausmarin A und B, spasmolytische Aktivität besitzen [vgl. Dhan Prakash et al., J. Chem. Soc. Chem. Commun. 1978, 281]. Außerdem gilt der wäßrige Extrakt aus Blättern von Clausena lansium (lour) Skeels in der chinesischen Volksmedizin als wirksames Leberschutzmittel und wird gegen akute und chronische Virushepatitis eingesetzt.

Aus diesem Extrakt konnten als Hauptbestandteile (±) 3(S*), 4((R*), 5(R*), 7(S*)-1-methyl-3-hydroxy-4-phenyl-5-(α-hydroxybenzyl)pyrrolidin-2-on (Clausenamid) und(±) 3(S*), 4(R*), 5(S*), 7(R*)-1-methyl-3-hydroxy-4-phenyl-5-(α-hydroxybenzyl)pyrrolidin-2-on (Neoclausenamid) isoliert werden.

Clausenamid zeigt im Tierversuch antiamnestische sowie vor cerebraler Hypoxie schützende Wirkung, während Neoclausenamid signifikant die durch Barbiturate induzierte Schlafzeit verkürzt. Clausenamid und Neoclausenamid unterdrücken außerdem den durch Tetrachlorkohlenstoff erhöhten Serumspiegel von Glutamin-Pyruvat-Transaminase in Mäusen.

Es wurden nun für weitere pharmakologische Studien größere Mengen an Clausenamid und Neoclausenamid benötigt, als durch das aufwendige Extraktionsverfahren gewonnen werden konnten. Somit war es notwendig, ein chemisches Verfahren zur Verfügung zu stellen, daß sowohl die Darstellung von Clausenamid und Neoclausenamid als auch ihre Derivatisierung ermöglicht. Ferner sollte sich das neue chemische Verfahren durch eine Steuerung der Stereoselektivität bzw. stereospezifisch in Richtung der bevorzugten Isomere mit der (3S*, 4R*, 5R*, 7S*) und (3S*, 4R*, 5S*, 7R*) Konfiguration in wesentlich besseren Ausbeuten als die bereits publizierten Verfahren auszeichnen.

Die vorliegende Erfindung betrifft nun ein Verfahren zur Herstellung von Clausenamid, Neoclausenamid und deren Derivate der allgemeinen Formel (I),

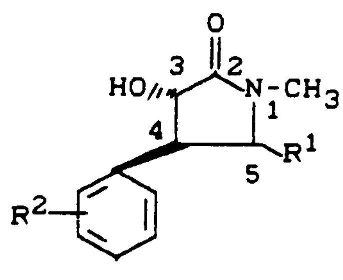

(I)

in welcher

$R^1$

- für die Gruppe der Formel

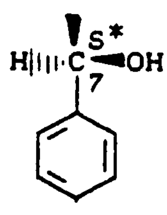

(Clausenamid 3S*, 4R*, 5R*, 7S*) steht, oder

- für die Gruppe der Formel

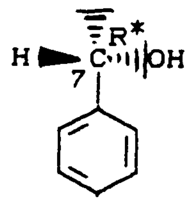

(Neoclausenamid 3S*, 4R*, 5S*, 7R*) steht, oder

- für die Gruppe der Formel

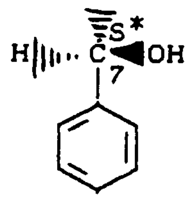

steht,

und

$R^2$

- für Wasserstoff, Fluor, Chlor oder Brom steht.

Die Verbindungen der allgemeinen Formel (I) können hergestellt werden, indem man Verbindungen der allgemeinen Formel (II),

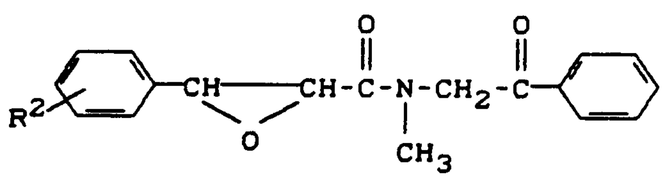
(II)

in welcher

$R^2$

- für Wasserstoff, Fluor, Chlor oder Brom steht,

in inerten organischen Lösemitteln in Anwesenheit einer Base unter Ausbildung der beiden Isomeren der Formeln (III) und (IV),

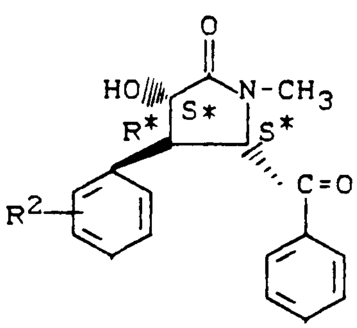
und
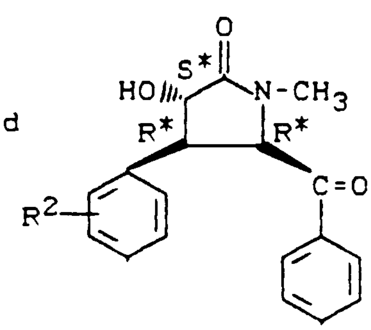

(III) (IV)

in welchen

$R^2$ die oben angegebene Bedeutung hat,

zunächst cyclisiert, und in einem weiteren Schritt entweder

a) Verbindungen der allgemeinen Formel (IV) stereospezifisch zu Verbindungen der allgemeinen Formel (I), in welcher $R^1$ für die Gruppe der Formel

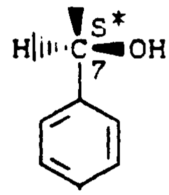

(Clausenamid)
steht,
reduziert, oder indem man
b) Verbindungen der allgemeinen Formel (III) je nach Wahl der Reaktionsbedingungen und der Reduktionsmittel zu Verbindungen der allgemeinen Formel (I), in welchen $R^1$ für die Gruppe der Formel

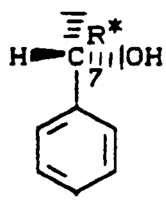

oder

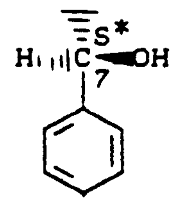

steht
reduziert, oder indem man
c) Verbindungen der allgemeinen Formel (III) mit 2,3-Dihydropyran zu Verbindungen der allgemeinen Formel (V),

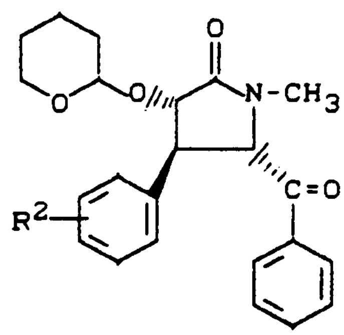

in welcher
$R^2$ die oben angegebene Bedeutung hat,
umsetzt, und anschließend stereoselektiv zu Verbindungen der allgemeinen Formel (I), in welcher $R^1$ für die Gruppe der Formel

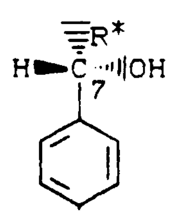

steht,

reduziert, und gegebenenfalls die Isomeren nach üblicher Methode chromatographisch trennt.

Das erfindungsgemäße Verfahren kann durch folgendes Formelschema erläutert werden:

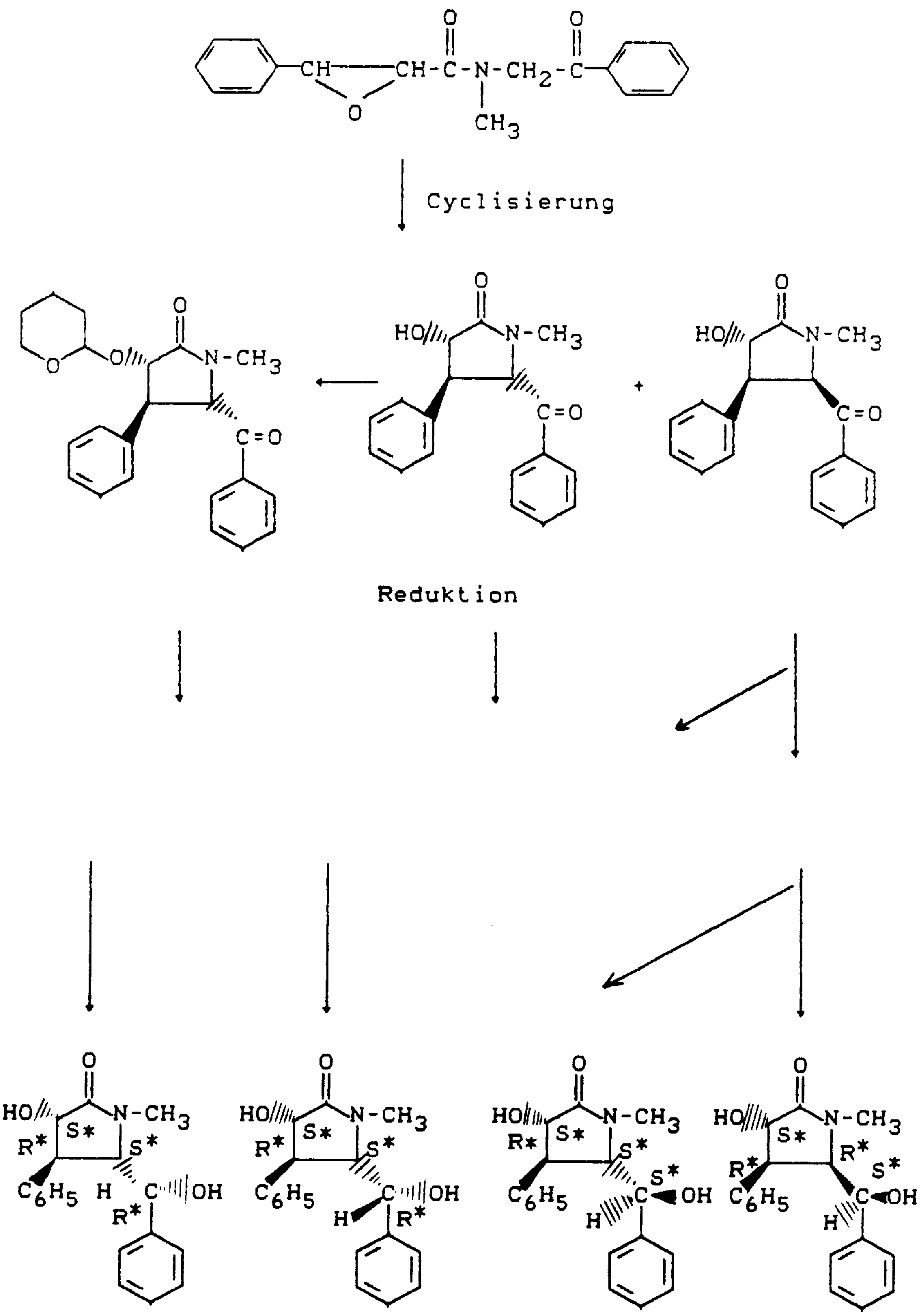


Neoclausenamid

Clausenamid

Als Lösemittel für die Cyclisierung eignen sich inerte organische Lösemittel wie Ether, beispielsweise Tetrahydrofuran, Diethylether oder Dioxan, oder Alkohole wie beispielsweise Methanol oder Ethanol, oder Halogenkohlenwasserstoffe wie beispielsweise Methylenchlorid oder Tetrachlorkohlenstoff, oder deren Gemische, gegebenenfalls auch mit Wasser. Bevorzugt sind Tetrahydrofuran, Methanol und Methylenchlorid.

Als Basen zur Cyclisierung eignen sich Alkalialkoholate, Alkaliamide oder Alkalihydride wie beispielsweise Natriumethanolat, Natriummethanolat, Kalium-, Natrium-oder Lithiumbutanolat, Lithium-, Natrium- oder Kaliumhydroxid, Natriumhydrid, Lithiumdiisopropylamid, Butyllithium oder Ammoniumhydroxide wie beispielsweise Tetramethylammoniumhydroxid. Bevorzugt sind Lithiumdiisopropylamid, Natriummethanolat, Lithiumhydroxid oder Tetramethylammoniumhydroxid.

Je nach Wahl der Base und der Reaktionsbedingungen kann die Produktbildung der Verbindungen der allgemeinen Formeln (III) und (IV) gesteuert werden. Die oben aufgeführten Alkalialkoholate und -hydroxide in Methanol begünstigen insgesamt die Cyclisierung, während unter den Bedingungen einer 2-Phasen-Transferreaktion, beispielsweise mit Tetramethylammoniumhydroxid als Base, bevorzugt die Verbindungen der allgemeinen Formel (IV) erhalten werden.

Die Reaktionstemperaturen liegen zwischen -70 °C und +40 °C. Bevorzugt wird zwischen -65 °C und +30 °C gearbeitet.

Bei der Cyclisierung werden pro Mol der Ausgangsverbindung 1 bis 5, bevorzugt 1 bis 2,5 Mol Base eingesetzt.

Die Cyclisierung kann bei normalem, aber auch bei erhöhtem oder erniedrigtem Druck durchgeführt werden. Im allgemeinen arbeitet man bei Normaldruck.

Die Reduktion der Ketofunktion zur Hydroxyfunktion erfolgt mit den üblichen Reduktionsmitteln wie metallische Hydride, komplexe Hydride oder mit organischen Aluminiumverbindungen. Bevorzugt sind Natriumborhydrid, Zinkborhydrid, Lithium-tri-sec-butyl-borhydrid oder Aluminiumpropylat.

Als Lösemittel eignen sich hierbei alle inerten organischen Lösemittel, die sich unter den Reaktionsbedingungen nicht verändern. Hierzu gehören bevorzugt Halogenkohlenwasserstoffe wie Methylenchlorid, Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure oder Dimethoxyethan. Ebenso ist es möglich, Gemische der genannten Lösemittel zu verwenden.

Als Katalysatoren bei der Reduktion werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Zur Vernichtung von überschüssigem Reduktionsmittel können Wasserstoffperoxid oder Basen wie Natriumhydroxid oder Wasser eingesetzt werden.

Die Reduktion wird bei einer Reaktionstemperatur von -10 °C bis +100 °C, bevorzugt bei 0 °C bis +80 °C, durchgeführt.

Die Reduktion kann bei normalem aber auch bei erhöhtem oder erniedrigtem Druck durchgeführt werden. Im allgemeinen arbeitet man bei Normaldruck.

Grundsätzlich muß bei der Reduktion der C(7)-Ketogruppe zur C(7)-Hydroxyfunktion in den Verbindungen der allgemeinen Formel (III) und (IV) beachtet werden, daß ein weiteres Chiralitätszentrum gebildet wird und somit 2 Diastereomere entstehen können.

Durch die Wahl des Reduktionsmittels kann die Reduktion stereospezifisch gesteuert werden, bzw. die Stereoselektivität erhöht werden. Beispielsweise erhält man bei der Reduktion der Verbindungen der allgemeinen Formel (IV) mit Natriumborhydrid/Tetrahydrofuran oder Natriumborhydrid/Methanol stereospezifisch das erwartete Isomer mit der Konfiguration ($3S_*$, $4R_*$, $5R_*$, $7S_*$) (Clausenamid und Derivate). Während man das Produktverhältnis der entstehenden Isomere bei der Reduktion der Ketone der allgemeinen Formel (III) durch die Wahl der Reaktionsbedingungen und des Reduktionsmittels bedingt steuern kann, ermöglicht die Überführung der Verbindungen der allgemeinen Formel (III) durch Schutz der Hydroxylfunktion, beispielsweise durch Umsetzung mit 2,3-Dihydropyran, in die Verbindungen der allgemeinen Formel (V) und anschließender Reduktion mit Natriumborhydrid/Tetrahydrofuran oder Lithium-tri-sec-butyl-borhydrid eine Erhöhung der Stereoselektivität in Richtung des gewünschten ($3S_*$, $4R_*$, $5R_*$, $7R_*$) (Neoclausenamid und Derivate) konfiguierten Isomers um > 90%.

Die Veretherung mit 2,3-Dihydropyran wird in einem der oben aufgeführten inerten Lösemitteln, bevorzugt in Methylenchlorid in Anwesenheit eines Katalysators, beispielsweise mit Pyridinium-p-Toluolsulfonsäure bei Raumtemperatur und Normaldruck durchgeführt.

Die Verbindungen der allgemeinen Formel (II) sind neu und können hergestellt werden, indem man [A] Verbindungen der allgemeinen Formel (VI),

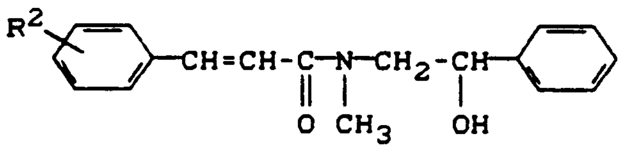

(VI)

in welcher
$R^2$ die oben angegebene Bedeutung hat,
zunächst nach üblicher Methode durch Oxidation der Hydroxygruppe in Verbindungen der allgemeinen Formel (VII),

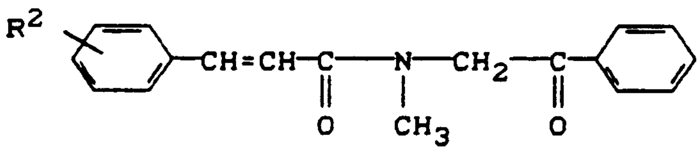

(VII)

in welcher
$R^2$ die oben angegebene Bedeutung hat,
überführt und in einem zweiten Schritt nach bekannter Methode eine Epoxidierung durchführt,
oder indem man
[B] Verbindungen der allgemeinen Formel (VIII),

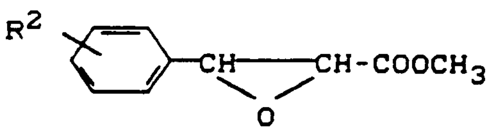

(VIII)

in welcher
$R^2$ die oben angegebene Bedeutung hat,
zunächst mit dem Amin der Formel (IX)

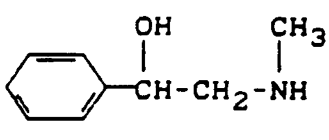

(IX)

nach üblicher Methode zu Verbindungen der allgemeinen Formel (X),

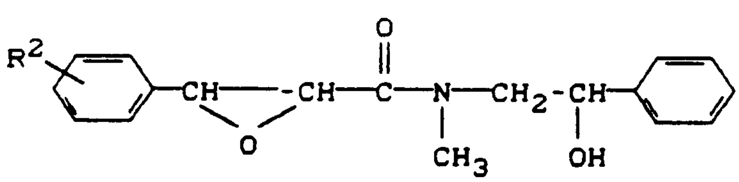

(X)

in welcher
$R^2$ die oben angegebene Bedeutung hat,
amidiert und in einem weiteren Schritt nach bekannter Methode die Hydroxygruppe oxidiert.

Der Reaktionsablauf kann durch folgendes Formelschema erläutert werden:

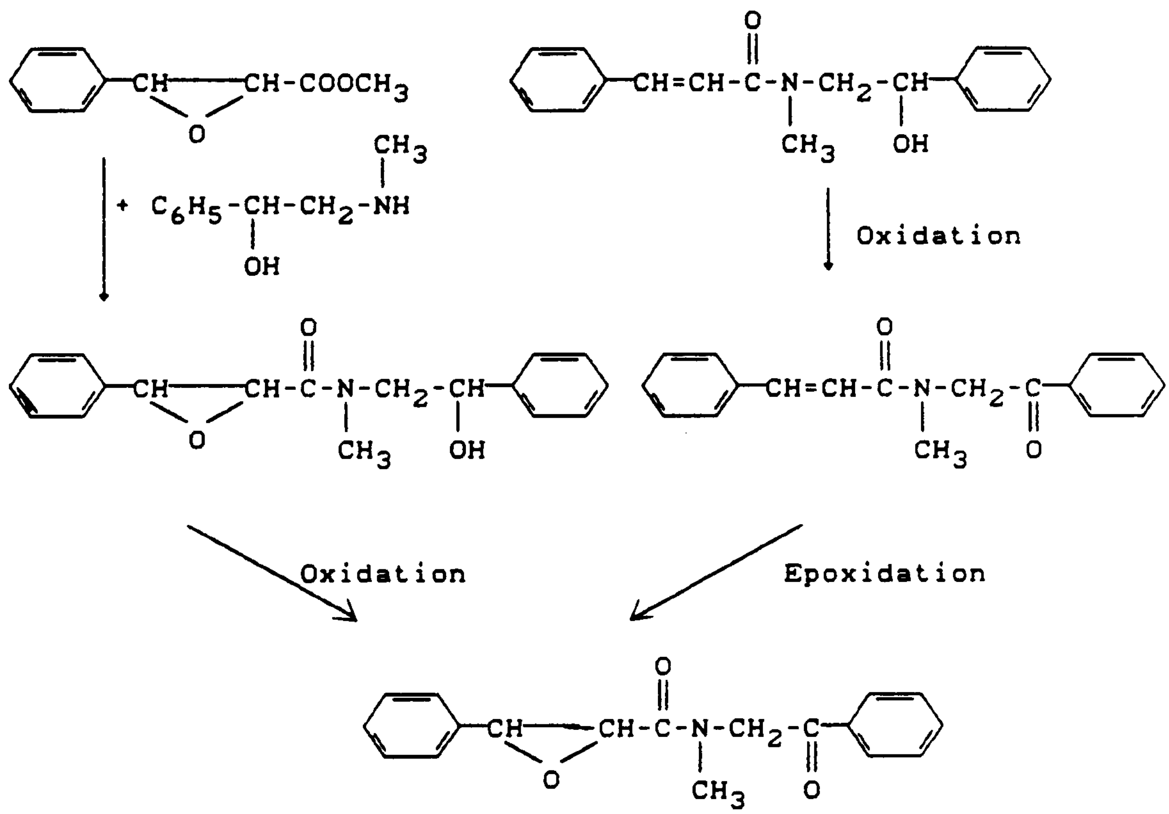


Als Lösungsmittel für die Oxidation der Hydroxygruppe eignen sich die üblichen organischen Lösemittel, die sich unter den Reaktionsbedingungen nicht verändern. Hierzu gehören vorzugsweise Kohlenwasserstoffe wie beispielsweise Benzol, Toluol, Xylol, Hexan, Cyclohexan oder Erdölfraktionen, Ether wie Diethylether, Tetrahydrofuran oder Dioxan, Alkohole wie beispielsweise Methanol, Ethanol oder Propanol, Halogenkohlenwasserstoffe wie beispielsweise Dichlormethan, Trichlormethan, Tetrachlormethan oder 1,2-Dichlorethan, Eisessig, Acetonitril oder Hexamethylphosphorsäuretriamid. Ebenso ist es möglich, Gemische der genannten Lösemittel zu verwenden.

Als Oxidationsmittel können organische oder anorganische Peroxoverbindungen wie beispielsweise Chlorperbenzoesäure oder Peroxoessigsäure, oder anorganische Oxide wie beispielsweise Chromoxide oder komplexe Chromoxide/Pyridin eingesetzt werden. Bevorzugt ist Chromoxid.

Die Reaktionstemperaturen können in einem Bereich von 0 °C bis +70 °C variiert werden. Bevorzugt wird zwischen +10 °C und +30 °C gearbeitet.

Die Oxidationen werden bei Normaldruck durchgeführt.

Bei der Durchführung der Oxidation werden pro Mol Ausgangsverbindung 1 bis 10 Mol, bevorzugt 5 Mol, Oxidationsmittel eingesetzt.

Die Epoxidierung verläuft ebenfalls in denen für die allgemeinen Oxidation aufgeführten inerten Lösemittel. Bevorzugt sind in diesem Fall Tetrachlorkohlenstoff und Methylenchlorid.

Als Oxidationsmittel für die Epoxidierung eignen sich organische Percursorsäuren wie beispielsweise Peroxoessigsäure oder Perbenzoesäure, oder Trifluoressigsäure, oder Oxide des Mangans, wie beispielsweise Mangandioxid. Bevorzugt sind p-Chlorbenzoesäure und Mangandioxid.

Die unter der allgemeinen Oxidation aufgeführten Angaben über die Menge an Oxidationsmittel, die Reaktionstemperatur, Druck und die Temperatur können auf die Epoxidierungsreaktion übertragen werden.

Die Amidierung verläuft in einem der oben aufgeführten inerten Lösemitteln, bevorzugt in Methanol.

Als Hilfsstoffe können tertiäre Amine wie beispielsweise 1,5-Diazabicyclo(4,3,0)non-5-en oder 1,8-Diazabicyclo(5,4,0)undec-7-en oder Dimethylaminopyridin eingesetzt werden.

Die Reaktionstemperaturen können zwischen -30 °C und 0 °C variiert werrden. Bevorzugt wird zwischen -20 °C und -10 °C gearbeitet.

Die Verbindungen der allgemeinen Formel (VI) sind an sich bekannt oder können nach bekannten Methoden hergestellt werden [vgl. Indian J. Chem., Sect. B, 19B (12), 1075-1076].

Die Verbindungen der allgemeinen Formel (VIII) sind an sich bekannt oder können nach üblicher Methode hergestellt werden [vgl. Beilstein, 18 (3), 4200].

Die Verbindungen der allgemeinen Formel (IX) sind an sich bekannt und können nach üblicher Methode hergestellt und eingesetzt werden [vgl. Helv. Chim. Acta 1978, 61 (6), 2237 -2240].

Beispielsweise seien genannt:
2-Ethylamino-1-phenylethanol
2-Methylamino-1-phenylethanol.

Es ist als überraschend zu bezeichnen, daß mit Hilfe des erfindungsgemäßen Verfahrens die richtig konfigurierten Verbindungen, wie Clausenamid (3S*, 4R*, 5R*, 7S*) und Neoclausenamid (3S*, 4R*, 5S*, 7R*) und ihre Derivate in guten Ausbeuten entstehen.

Die Verbindungen mit $R^2$ = H sind mit dem aus dem Pflanzenextrakt gewonnenen Clausenamid und Neoclausenamid identisch. Mit dem neuen Verfahren können im Vergleich zum Extraktionsverfahren wesentlich größere Mengen in kürzerer Zeit und mit weniger Aufwand zur Verfügung gestellt werden.

Herstellungsbeispiele

Beispiel 1

N-Methyl-N-phenacyl-zimtsäureamid

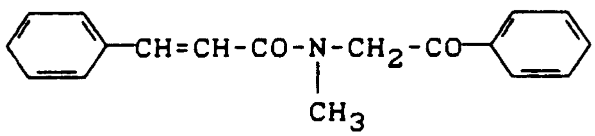

Es wird eine Chromoxid-Lösung durch Zugabe von 9,0 g (0,09 mol) Chromoxid zu einer Mischung von 14,25 g (0,18 mol) trockenem Pyridin und 210 ml Methylenchlorid hergestellt. Anschließend wird 1 h bei Raumtemperatur gerührt und dabei eine rote Lösung erhalten. Zu dieser Lösung werden 4,2 g (0,015 mol) N-Methyl-N-(β-hydroxy-β-phenyl)-ethyl-zimt-amid in 40 ml Methylenchlorid zugegeben und 15 min gerührt. Die Reaktionslösung wird schnell auf eine kurze Säule (Silicagel 25 x 2 cm) gegeben und diese mit 200 ml Methylenchlorid gewaschen. Das Filtrat wird bis zur Trockene eingeengt. Man erhält 3,4 g eines gelben Feststoffes, der aus Benzol umkristallisiert wird.

| | | | |
|---|---|---|---|
| Ausbeute: | 3,1 g | F °C: | 141-142,5 °C |
| | 0,1 g | F °C: | 138-141 °C (aus Mutterlauge) |

Summenformel: $C_{18}H_{17}NO_2$

| | | | | |
|---|---|---|---|---|
| Elementaranalyse: | kal.% = | C 77,42 | H 6,09 | N 5,02 |
| | gef.% = | C 77,44 | H 6,12 | N 4,90 |

Beispiel 2

N-Methyl-N-(β-hydroxy-β-phenyl)-ethyl-3-phenylglycidamid

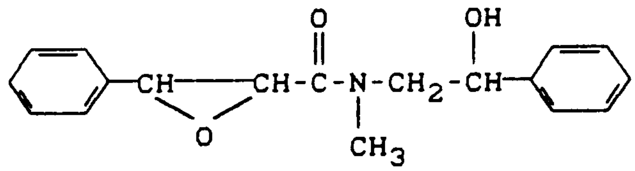

a) 35,7 g (0,2 mol) Methyl-3-phenylglycidat in 50 ml Methanol und 43,6 g (0,28 mol) N-Methyl-β-hydroxy-β-phenyl-ethylamin in 50 ml Methanol werden separat auf -16 °C abgekühlt und anschließend unter Zugabe von 1,0 g 4-Dimethylaminopyridin zusammengegeben. Bei einer Temperatur von -16 °C wird 5 Tage stehen gelassen und jeden Tag unter Rühren 20 ml Wasser zugegeben. Nach 5 Tagen wird der Rückstand abfiltriert und bis zum Ausflocken mit eisgekühltem Methanol gewaschen. Man erhält einen weißen Feststoff.
Ausbeute: 25,9 g (43,5% der Theorie)
$R_f$: 0,35 ($SiO_2$-Platte, Laufmittelsystem Chloroform/Methanol 100:3)
F °C: 143-146 °C

b) Zu einer Lösung von 33,2 g (0,22 mol) N-Methyl-β-hydroxy-β-phenylethylamin in 50 ml Methanol werden 2,6 g einer 25 - 28%igen Natriummethanolat/Methanol-Lösung gegeben. Es wird auf -16 °C gekühlt und eine kalte Lösung (-16 °C) von 35,6 g (0,2 mol) Methyl-3-phenylglycidat in 50 ml Methanol zugegeben. Die Lösung wird bei -16 °C 24 Stunden stehen gelassen. Der abfiltrierte Rückstand wird mit eisgekühltem Methanol und Ether gewaschen. Weiteres Produkt wird aus der Mutterlauge gewonnen. Man erhält einen weißen Feststoff.
Ausbeute: 27,5 g (46,3% der Theorie)
F °C: 150-151 °C

Beispiel 3

N-methyl-N-phenacyl-3-phenylglycidamid

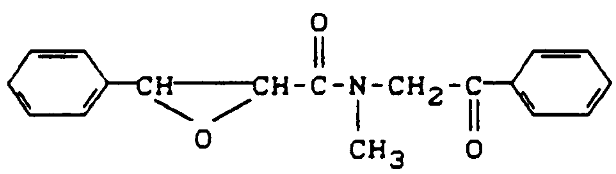

a) 2,4 g (0,0086 mol) der Verbindung aus Beispiel 1 und 8,6 g (0,04 mol) m-Chlorperbenzoesäure werden in 170 ml Chloroform gelöst und 2 Tage bei Raumtemperatur stehen gelassen. Der Niederschlag wird abfiltriert und das Filtrat wird nacheinander mit Natriumsulfitlösung (10%), Natriumcarbonatlösung (10%) und Wasser gewaschen und über wasserfreiem Natriumsulfat getrocknet. Das Lösemittel wird entfernt, der Rückstand in 6 ml Benzol/trockenem Ether (1:1) gelöst und im Eisschrank gekühlt. Nach Abtrennen von nicht umgesetztem Ausgangsmaterial wird abfiltriert. Der ölige Rückstand wird chromatographisch gereinigt.
Ausbeute: 1,25 g (49,2% der Theorie)
$^1H$-NMR (90 MHz, $CDCl_3$): δ = 3,09 (s) + 3,21 (s, 3H, $NCH_3$); 3,49 (d) + 3,88 (d, J = 2,7 Hz, 1H); 4,04 (d) + 4,15 (d, J = 2,7 Hz, 1H); 4,82, 5,04 (AB, J = 18 Hz) + 4,96 (s, 2H); 7,30 - 7,8 (m, 8H ); 8,01 (dd, J = 1,8 Hz, 8 Hz) + 8,06 (dd, J = 2,7 Hz, 8 Hz, 2H).

b) Zu einer Lösung von 5,94 g (0,02 mol) der Verbindung aus Beispiel 2 werden 36 g aktiviertes Mangandioxid unter kräftigem Rühren zugeben. Es wird 1,5 h gerührt bis bei der dünnschichtchromatographischen Überprüfung kein Ausgangsmaterial mehr nachweisbar ist. Es wird vom Mangandioxid abfiltriert und mit Methylenchlorid gewaschen. Die vereinigten Filtrate werden zunächst mit 20 ml einer

15% Natriumhydrogensulfit-Lösung und 20 ml einer gesättigten Natriumchlorid-Lösung gewaschen und über wasserfreiem Natriumsulfat getrocknet. Man erhält einen viskosen transparenten Rückstand, der sich beim Stehenlassen verfestigt. Durch Umkristallisieren erhält man weiße Nadeln.
Ausbeute: 4,42 g (75% der Theorie)
F ° C: 76,5 - 79 ° C
Nach einer zweiten Umkristallisation wird ein Schmelzpunkt von 78,5 - 80 ° C festgestellt.
$^1$H-NMR ($CDCl_3$): δ = 3,12 (s) + 3,26 (s); (3H, $NCH_3$); 3,52 (d) + 3,85 (d, 1H, J = 2 Hz); 4,07 (d), 4,19 (d, 1H, J = 2 Hz); 4,88 (d); 5,10 (d); (2H, J = 18 Hz, $PhCOCH_2$); 7,30 - 8,16 (m, 10H).

In Analogie zu den Vorschriften der Beispiele 2 und 3 können die in der folgenden Tabellen 1 und 2 aufgeführten Verbindungen hergestellt werden.

Tabelle 1

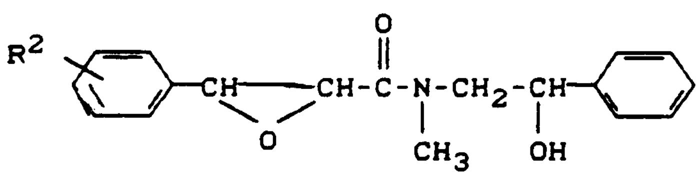

| Bsp.-Nr. | $R^2$ | F° C | Ausbeute |
|---|---|---|---|
| 4 | m-Br | 145-146° C | 38,4 g |
| 5 | m-Cl | 131-132° C | 32,4 g |
| 6 | m-F | 134-135° C | 29,5 g |
| 7 | p-F | 150-152° C | 34,9 g |
| 8 | p-Br | 154-156° C | 31,4 g |

Tabelle 2

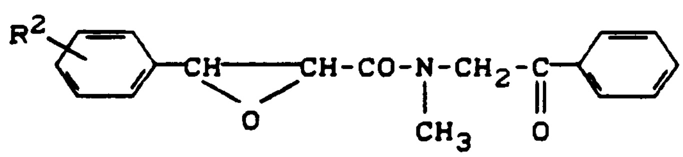

| Bsp.-Nr. | $R^2$ | F° C | Ausbeute |
|---|---|---|---|
| 9 | m-Br | 117-119° C | 71,3% |
| 10 | m-Cl | 85-87° C | 67,1% |
| 11 | m-F | 81-83° C | 72,9% |
| 12 | p-F | 99-101° C | 85,7% |
| 13 | p-Br | 154-156° C | 74,2% |

Beispiel 14 und Beispiel 15

(±) 3(S*), 4(R*), 5(R*)-1-methyl-3-hydroxy-4-phenyl-5-benzoyl-pyrrolidin-2-on (14) und
(±) 3(S*), 4(R*), 5(S*)-1-methyl-3-hydroxy-4-phenyl-5-benzoyl-pyrrolidin-2-on (15)

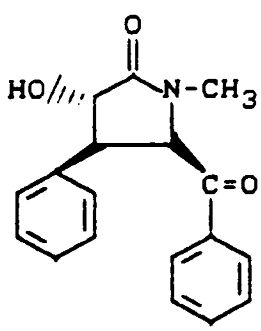

(14)

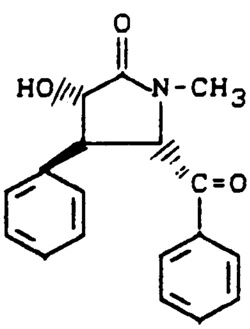

(15)

a) Zu einer Lösung von 4 mmol Lithiumdiisopropylamid in 5 ml Tetrahydrofuran werden bei -72 °C 490 mg (1,66 mmol) der Verbindung aus Beispiel 3 in 10 ml Tetrahydrofuran unter Rühren und Kühlen zugetropft. Es wird über 4 h gekühlt und gerührt, bis kein Ausgangsmaterial mehr nachweisbar ist. Dann werden 100 ml Wasser langsam zugegeben. Tetrahydrofuran wird im Vakuum entfernt. Man erhält 680 mg eines braunen halbfesten Rohproduktes, das chromatographisch (Chromatotron) gereinigt wird. Man erhält die Verbindung aus Beispiel 14 mit einem Schmelzpunkt von 193-201 °C. Nach Umkristallisation liegt der Schmelzpunkt bei 208 - 210 °C. Das Produkt ist identisch mit der Verbindung, die man durch Oxidation von natürlichem Clausenamid erhält. Ferner zeigen die Schmelzpunkte der Mischung keine Erniedrigung.
Summenformel: $C_{18}H_{17}NO_3$

| | | | | |
|---|---|---|---|---|
| Elementaranalyse: | kalk.:<br>gef. : | C 73,22<br>C 73,27 | H 5,76<br>H 5,67 | N 4,75<br>N 4,70 |

$^1$H-NMR ($CDCl_3$): δ = 2,92 (s, 3H, $NCH_3$); 3,92 (t, J = 8,5 Hz, 1H, $C_4$H); 3,40 (br, s, 1H, austauschbar mit $D_2O$); 4,93 (d, J = 8,5 Hz, 1H, $C_3$-H); 5,50 (d, J = 8,5 Hz, 1H, $C_5$-H); 7,04-7,84 (m, 10H, ArH).

Durch die chromatographische Auftrennung erhält man außerdem die Verbindung aus Beispiel 15 mit dem Schmelzpunkt von 198 - 200 °C.

$^1$H-NMR ($CDCl_3$): δ = 2,97 (s, 3H, $NCH_3$); 3,35 (t, J = 7,2 Hz, 1H, $C_4$-H); 3,70 (br, s, 1H, austauschbar mit $D_2O$); 4,58 (d, J = 7,2 Hz, 1H, $C_3$-H); 5,18 (d, J = 7,2 Hz, 1H, $C_5$-H); 7,04 - 7,84 (m, 10H, ArH).

Die Gesamtausbeute von Beispiel 14 und Beispiel 15 beträgt gesamt 30% der Theorie in einem Verhältnis von 1:2.

b) Eine Lösung von 2,95 g (10 mmol) der Verbindung aus Beispiel 3 in 70 ml Methanol wird zu einer Lösung von Natriummethanolat (in situ hergestellt aus 1,15 g (5 mmol) Natrium und 100 ml absolutem Methanol unter Stickstoffatmosphäre) unter Rühren zugegeben. Es wird 30. min gerührt, mit 3,0 g Eisessig angesäuert und anschließend konzentriert. Der Rückstand wird in Methylenchlorid gelöst und auf einer Säule ($SiO_2$) chromatographisch aufgetrennt. Man erhält 0,59 g (20% der Theorie) der Verbindung aus Beispiel 14 mit dem Schmelzpunkt 210 - 214 °C (umkristallisiert aus Ethylacetat) und 1,59 g (53% der Theorie) der Verbindung aus Beispiel 15 mit dem Schmelzpunkt 180 - 182 °C (ebenfalls aus Essigester umkristallisiert).

c) 8,8 g (28,8 mmol) der Verbindung aus Beispiel 3 werden in 100 ml Methylenchlorid gelöst und 50 ml einer 1%igen Tetramethylammoniumhydroxid-Lösung zugegeben. Die Lösung wird 24 h bei Raumtemperatur gerührt. Die organische Phase wird abgetrennt und die wäßrige Phase mit Methylenchlorid extrahiert. Die organischen Phasen werden vereinigt und mit gesättigter Natriumbicarbonatlösung und Natriumchloridlösung gewaschen. Nach dem Entfernen des Lösemittels erhält man 8,2 g eines Feststoffs. Dieser wird aus Ethylacetat umkristallisiert und man erhält 6,1 g der Verbindung aus Beispiel 15. Unter Berücksichtigung der Nachfällung aus der Mutterlauge erhält man insgesamt eine Gesamtausbeute von 80,6% der Theorie.

Nach säulenchromatographischer Trennung und Umkristallisation erhält man 1,55 g (17,6% der Theorie) der Verbindung aus Beispiel 14 mit dem Schmelzpunkt 213 - 215 °C und 4,8 g (54,5% der Theorie) der Verbindung aus Beispiel 15 mit dem Schmelzpunkt 182 - 183 °C.

d) Eine Lösung von 0,168 g (4 mmol) Lithiumhydroxidlösung in 40 ml Wasser wird auf 3 °C gekühlt und 1,18 g (4 mmol) der Verbindung aus Beispiel 3 in 20 ml Methanol unter Rühren zugetropft. Anschließend wird ca. 5 h bei 3 °C gerührt. Nach einer Nacht im Gefrierschrank erhält man 0,96 g eines ausgefallenen weißen Feststoffs, der aus der Lösung abfiltriert wird und weitere 0,21 g aus dem Filtrat.

Nach chromatographischer Reinigung und Umkristallisation erhält man 462 mg (39% der Theorie) der Verbindung aus Beispiel 14 vom Schmelzpunkt 203-205 °C und 357 mg (30% der Theorie) der Verbindung aus Beispiel 15.

Die Cyclisierung zu den Verbindungen der Beispiele 14 und 15 kann auch unter anderen Reaktionsbedingungen ablaufen, die in Tabelle 3 aufgeführt sind.

| Lösemittel | Base | Temp. °C | Zeit h | Ausbeute 14 + 15 | Verhältnis 14:15 |
|---|---|---|---|---|---|
| Dioxan | LDA(in Hexan) | -65 | 5 | 21,4 | 1//2,5 |
| Benzol | BuLi(in Hexan) | 10 | 3 | 22,7 | 1/2,6 |
| Benzol | NaH | 10 | 4 | 18,8 | 1/3 |
| t-BuOH | t-BuOLi | 25-30 | 2 | 23,2 | 1/3,6 |
| t-BuoOH | t-BuONa | 25-30 | 1,5 | 21,2 | 1/3,4 |
| t-BuOH | t-BuOK | 25-30 | 2/3 | 21,4 | 1/2,8 |
| $Ch_3OH$ | $CH_3OLi$ | 2-4 | 3,5 | 83,4 | 1/1,9 |
| $CH_3OH$ | $CH_3OK$ | 2-4 | 4 | 75,5 | 1/2,1 |
| $CH_3OH$ | $LiOH.H_2O$ | 2-4 | 5 | 86,8 | 1/1,7 |
| $CH_3OH$ | NaOH | 2-4 | 5 | 85,1 | 1/2 |
| $CH_3OH$ | KOH | 2-4 | 5 | 80,1 | 1/1,9 |
| $CH_3OH/H_2O$ | $Me_4N^+OH^-$ | 13 | 7 | 85 | 1/1,9 |
| $CH_2Cl_2/H_2O$ | KOH | 15 | 12 | 77 | 1/2,1 |
| $Et_2O/H_2O$ | KOH | 15 | 8 | 77 | 1/1,2 |
| $Et_2O/THF/H_2O$ | LiOH | 6-8 | 2,5 | 80 | 1/1 |

Die in Tabelle 4 aufgeführten Beispiele werden in Analogie zu den Vorschriften für die Beispiele 14 und 15 hergestellt:

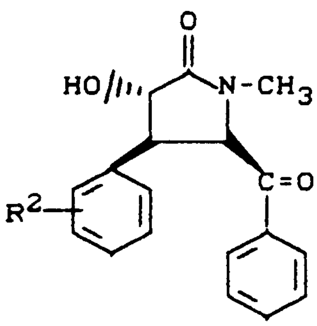

(14)

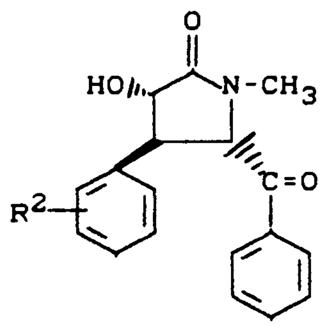

(15)

| Bsp.-Nr. | $R^2$ | Gesamt-Ausbeute (%) | F ° C | IR |
|---|---|---|---|---|
| 16 | m-Br | 74,7 | 187-188 (cis) | 3250 (OH)<br>1700 (Ph-CO)<br>1670 (-NCO) |
| 17 | m-Br | 74,7 | 166-168 (trans) | |
| 18 | m-Cl | 74,8 | 179-180 (cis) | 3240 (OH)<br>1680 (N-CO)<br>(Ph-CO) |
| 19 | m-Cl | 74,8 | 160-162 (trans) | |
| 20 | m-F | 73 | 178-180 (cis) | 3300 (OH)<br>1670 (-NCO)<br>1700 (Ph-CO) |
| 21 | m-F | 73 | 155-157 (trans) | |
| 22 | p-F | 74,6 | 180-183 (cis) | 3250 (OH)<br>1675 (-NCO)<br>1690 (Ph-CO) |
| 23 | p-F | 74,6 | 157-159 (trans) | |
| 24 | p-Br | 76,6 | 173-175 (cis) | 3250 (OH)<br>1675 (-NCO)<br>1695 (Ph-CO) |
| 25 | p-Br | 76,6 | 145-147 (trans) | |

Beispiel 26

Clausenamid

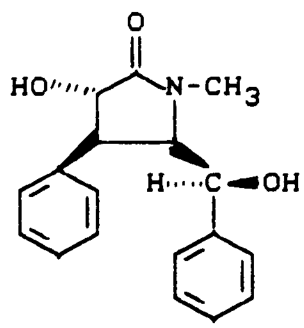

Zu einer Lösung von 74 mg (0,25 mmol) der Verbindung aus Beispiel 14 in 10 ml trockenem Methanol werden 15 mg (0,4 mmol) Natriumborhydrid unter Rühren zugetropft. Es wird solange gerührt (40 min) bis kein Ausgangsmaterial dünnschichtchromatographisch mehr nachweisbar ist. Das Methanol wird im Vakuum entfernt und der Rückstand auf pH 5-6 durch Ansäuern mit verdünnter (5%iger) Salzsäure eingestellt. Der Rückstand wird abfiltriert. Weiteren weißen Feststoff erhält man aus dem Methylenchloridextrakt der sauren wäßrigen Phase. Der weiße Feststoff liegt in einer Menge von 62 mg (83,7% der Theorie) vor und zeigt einen Schmelzpunkt von 237 - 239 ° C. Nach der Umkristallisation liegt der Schmelzpunkt bei 244 - 246 ° C. Es wird keine Schmelzpunktserniedrigung beobachtet, wenn man das synthetische Produkt mit dem natürlich gewonnenen Produkt vermischt.

Summenformel: $C_{18}H_{19}NO_3$

| Elementaranalyse: | kal.:<br>gef.: | C 72,70<br>C 72,57 | H 6,44<br>H 6,46 | N 4,71<br>N 4,50 |
|---|---|---|---|---|

$^1$H-NMR (DMSO-d, 90 MHz): δ = 3,05 (s, 3H, $NCH_3$); 3,60 (m, 1H, $C_4$-H); 3,88 (m, 1H, $C_3$-H); 4,32 (m, 1H, $C_5$-H); 4,67 (d, 1H, J = 3 Hz, $C_7$-H); 5,42 (m, 1H, OH); 6,55 - 7,25 (m, 10H, ArH).

Analog der Vorschrift für Beispiel 26 werden die in der Tabelle 5 aufgeführten Verbindungen hergestellt:

Tabelle 5

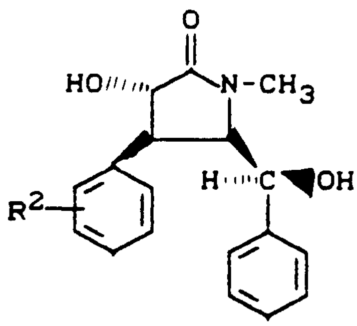

| Bsp.-Nr. | $R^2$ | F° C | Ausbeute | IR |
|---|---|---|---|---|
| 27 | m-Br | 215-217 | 85 | 1685 (Amid) |
| 28 | m-Cl | 214-216 | 74 | 1665 (Amid) |
| 29 | p-F | 251-253 | 75 | 1680 (Amid) |
| 30 | m-F | 225-227 | 84 | 1675 (Amid) |
| 31 | p-Br | 246-248 | 84 | 1660 (Amid) |

Beispiel 32

(±) 3(S*), 4(R*), 5(S*)-1-methyl-3-o-tetrahydropyranyl-4-phenyl-5-benzoyl-pyrrolidin-2-on

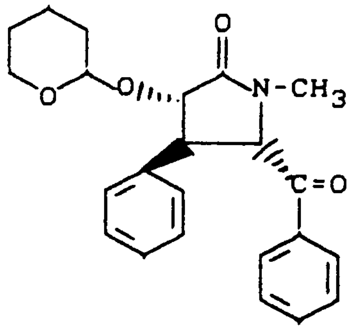

0,89 g (3 mmol) der Verbindung aus Beispiel 15 werden in 35 ml Methylenchlorid gelöst und anschließend mit 760 mg 2,3-Dihydropyran und 75 ml Pyridinium-p-tolylsulfonat umgesetzt. Die Reaktionslösung wird über Nacht bei Raumtemperatur gerührt. Anschließend werden 15 ml Methylenchlorid zugefügt und die Lösung wird mit einer gesättigten Natriumchlorid-Lösung gewaschen und über wasserfreiem Magnesiumsulfat getrocknet. 1,6 g weißer Feststoff werden nach Entfernung des Lösemittels erhalten. Nach chromatographischer Reinigung erhält man 1,05 g (92% der Theorie) der Titelverbindung mit dem Schmelzpunkt 165-168 ° C. Es wird aus Methylenchlorid/Diethylether umkristallisiert.
Die Kristalle haben einen Schmelzpunkt von 173-175 ° C.
Summenformel: $C_{23}H_{25}NO_4$

| | | | | |
|---|---|---|---|---|
| Elementaranalyse: | kal.: | C 72,80 | H 6,64 | N 3,69 |
| | gef.: | C 72,97 | H 6,66 | N 3,73 |

$^1$H-NMR ($CDCl_3$, 90 MHz): δ = 1,20 - 2,0 (m, 6H); 2,93 (s, 3H, $NCH_3$); 3,10 - 3,48 (m, 3H); 4,46 + 4,64 (d + d, 1H, J = 6,3 Hz, $C_3$-H); 4,97 + 4,95 (d + d, 1H, J = 5,5 Hz, $C_5$-H); 5,17 + 4,10 (m + m, 1H); 7,10 - 7,70 (m, 10H, ArH).

Beispiel 33 und Beispiel 34

(±) 3(S*), 4(R*), 5(S*), 7(R*)-1-methyl-3-hydroxy-4-phenyl-5-(α-hydroxybenzyl)pyrrolidin-2-on (Neoclausenamid)

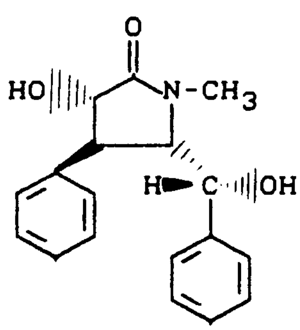

(38)

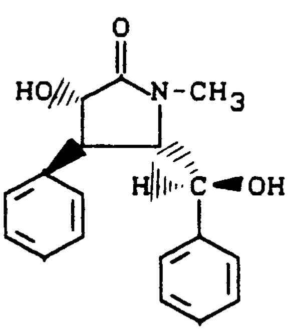

(39)

Lithium-tri-sec-butylborhydrid-Reduktion

a) 100 mg der Verbindung aus Beispiel 15 in 2 ml trockenem Tetrahydrofuran werden auf 10 °C gekühlt und 2 ml (2 mmol) einer Lithium-tri-sec-butyl-borhydrid/Tetrahydrofuran-Lösung zugegeben. Die Reaktionslösung wird bei -10 °C 1 h gerührt. Anschließend werden 1 ml 30%ige Wasserstoffperoxidlösung und 1 ml 2 N Natriumhydroxid-Lösung zugegeben. Es wird 3 mal mit je 10 ml Methylenchlorid extrahiert. Die Extrakte werden mit gesättigter Natriumbicarbonatlösung und mit Wasser gewaschen und anschließend über trockenem Natriumsulfat getrocknet. Das Lösemittel wird bis fast zur Trockene entfernt. Anschließend wird mit 2 ml Diethylether versetzt. Man erhält 91 mg eines weißen Feststoffs.
Dünnschichtchromatographisch zeigen sich 2 Produkte mit den $R_f$-Werten (Silicagelplatte, Essigester) 0,23 und 0,35, die dem Neoclausenamid (Beispiel 33) und dem Isomer (Beispiel 34) zugeordnet werden können.
Aus dem Dünnschichtchromatogramm und der Verschiebung der Signale der Gruppe $-N-CH_3$ im $^1H$-NMR ($\delta$ = 3,15; 2,89) läßt sich das Verhältnis der Produktbildung der Beispiele 33 und 34 zu 3:1 bestimmen.
b) Eine Lösung von 379 mg (1,0 mmol) der Verbindung aus Beispiel 32 in 8 ml Tetrahydrofuran wird mit 3 ml (3 mmol) Lithium-tri-sec-butyl-borhydrid in Tetrahydrofuran bei -15 °C versetzt. Die Reaktionslösung wird bei -10 °C bis -15 °C 40 min gerührt. Anschließend werden 5 ml Wasser zugegeben und die Lösung mit 1 ml 3,7 N Schwefelsäure angesäuert. Es wird 5 h bei Raumtemperatur gerührt und mit 50 ml Chloroform extrahiert. Die Chloroformlösung wird nacheinander mit 2 N Natriumhydroxidlösung, Natriumbicarbonatlösung und gesättigter Natriumchloridlösung gewaschen bis die Lösung neutral ist. Die Lösung wird getrocknet und das Lösemittel entfernt. Der Rückstand wird im System Ethanol/Wasser (1:1),das 1 Tropfen 3,7 N Schwefelsäure enthält, umkristallisiert. Man erhält 240 mg (51% der Theorie) eines Feststoffs vom Schmelzpunkt 209-212 °C (Schmelzpunkt des natürlichen Neoclausenamids 205-206 °C).

$^1H$-NMR ($CDCl_3$, 90 MHz): $\delta$ = 2,0 (br.s, 2H); 3,08 (s, 3H, $NCH_3$); 3,15 (t, 1H, J = 3 Hz, $C_4$-H); 3,74 (t, 1H, J = 3 Hz, $C_5$-H); 4,13 (d, 1H, J = 3 Hz, $C_3$-H); 5,17 (d, 1H, J = 3 Hz, $C_7$-H); 6,64 - 7,29 (m, 10H, ArH).

Die Ergebnisse der Reduktion der Verbindungen aus den Beispielen 15 und 32 unter verschiedenen Reaktionsbedingungen werden durch die in der Tabelle 6 aufgeführten Beispiele dokumentiert.

Tabelle 6

| Ausgangs-.verbdg. | Lösemittel | Temp. (°C) | Verhältnis (33)/(34) | Ausbeute (%) |
|---|---|---|---|---|
| 15 | MeOH | RT | 1,2/1 | 60 |
| 32 | MeOH | RT | 10/1 | 54 |
| 15 | THF | RT | 1/1 | 54 |
| 32 | THF | RT | 10/1 | 41 |
| 15 | $CH_2Cl_2$ | 0-5 | 1/2 | 91 |
| 32 | $CH_2Cl_2$ | 0-5 | 8/1 | 77 |
| 15 | DME | 0 | 1/1,2 | |
| 15 | THF | -15 | 2,8/1 | 80-90 |
| 32 | THF | -15 | 10/1 | 81 |
| 15 | i-PrOH | 80 | 1/20 | 80-90 |
| 32 | i-PrOH | 80 | 1/1 | 57 |
| 15 | $Et_2O$ | RT | 1,5/1 | 70 |

Anlage zum experimentellen Teil

DME = Dimethoxyethan
LDA = Lithiumdiisopropylamid
tBUOH = tert.Butanol
THF = Tetrahydrofuran

$CH_2Cl_2$ = Methylenchlorid
i-PrOH = Isopropanol
MeOH = Methanol

## Patentansprüche

1. Verfahren zur Herstellung von Clausenamid, Neoclausenamid und deren Derivate der allgemeinen Formel,

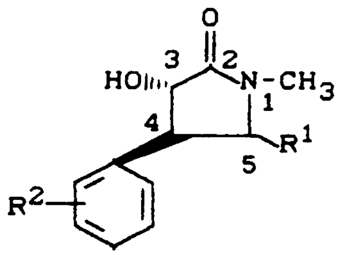

(I)

in welcher
$R^1$
- für die Gruppe der Formel

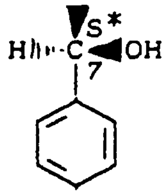

(Clausenamid 3S*, 4R*, 5R*, 7S*)
steht, oder
- für die Gruppe der Formel

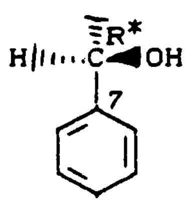

(Neoclausenamid 3S*, 4R*, 5S*. 7R*)
steht, oder
- für die Gruppe der Formel

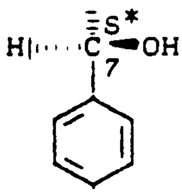

steht,
und
$R^2$

- für Wasserstoff, Fluor, Chlor oder Brom steht,

dadurch gekennzeichnet, daß man Verbindungen der allgemeinen Formel,

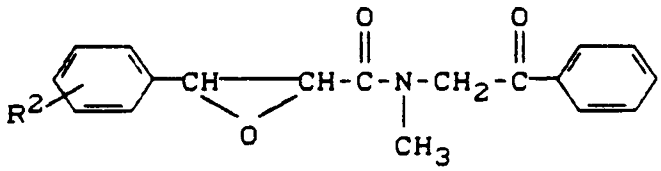

(II)

in welcher

$R^2$
- für Wasserstoff, Fluor, Chlor oder Brom steht,

in inerten organischen Lösemitteln in Anwesenheit einer Base unter Ausbildung der beiden Isomeren der Formeln,

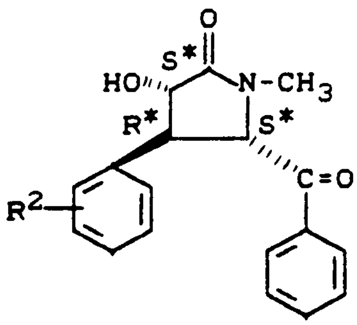

und

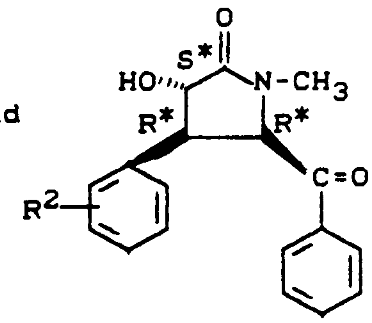

(III) (IV)

in welchen

$R^2$ die oben angegebene Bedeutung hat,

zunächst cyclisiert, und in einem weiteren Schritt entweder

a) Verbindungen der allgemeinen Formel (IV) stereospezifisch zu Verbindungen der allgemeinen Formel (I), in welcher $R^1$ für die Gruppe der Formel

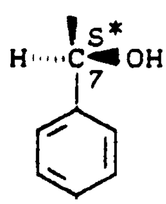

(Clausenamid)

steht,

reduziert,

oder daß man

b) Verbindungen der allgemeinen Formel (III) je nach Wahl der Reaktionsbedingungen und der Reduktionsmittel zu Verbindungen der allgemeinen Formel (I), in welchen $R^1$ für die Gruppe der Formel

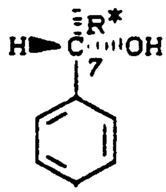

oder

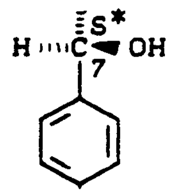

steht
reduziert, oder indem man
c) Verbindungen der allgemeinen Formel (III) mit 2,3-Dihydropyran zu Verbindungen der allgemeinen Formel,

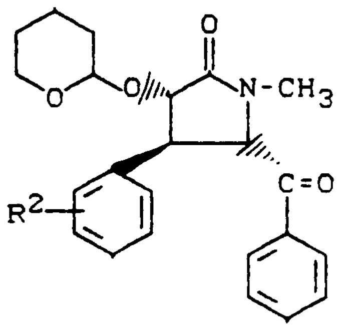

(V)

in welcher
$R^2$ die oben angegebene Bedeutung hat,
umsetzt, und anschließend stereoselektiv zu Verbindungen der allgemeinen Formel (I), in welcher $R^1$ für die Gruppe der Formel

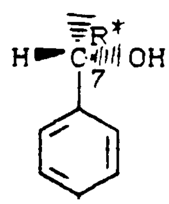

steht,
reduziert, und gegebenenfalls die Isomeren nach üblicher Methode chromatographisch trennt.

**2.** Verfahren nach Anspruch 1, dadurch gekennzeichnet, daß man als Lösemittel für die Cyclisierung inerte organische Lösemittel wie Ether, beispielsweise Tetrahydrofuran, Diethylether oder Dioxan, oder Alkohole wie beispielsweise Methanol oder Ethanol, oder Halogenkohlenwasserstoffe wie beispielsweise Methylenchlorid oder Tetrachlorkohlenstoff, oder deren Gemische, gegebenenfalls auch mit Wasser, einsetzt.

**3.** Verfahren nach Anspruch 1 und 2, dadurch gekennzeichnet, daß man als Basen zur Cyclisierung Alkalialkoholate, Alkaliamide oder Alkalihydride wie beispielsweise Natriumethanolat, Natriummethanolat, Kalium-, Natrium-oder Lithiumbutanolat, Lithium-, Natrium- oder Kaliumhydroxid, Natriumhydrid, Lithiumdiisopropylamid, Butyllithium oder Ammoniumhydroxide wie beispielsweise Tetramethylammoniumhydroxid einsetzt.

**4.** Verfahren nach den Ansprüchen 1 bis 3, dadurch gekennzeichnet, daß man die Cyclisierung unter den Bedingungen einer 2-Phasen-Transferreaktion durchführt.

**5.** Verfahren nach den Ansprüchen 1 bis 4, dadurch gekennzeichnet, daß die Reduktion der Ketofunktion zur Hydroxyfunktion mit den üblichen Reduktionsmitteln wie metallische Hydride, komplexe Hydride

oder mit organischen Aluminiumverbindungen durchgeführt wird.

6. Verfahren nach den Ansprüchen 1 bis 5, dadurch gekennzeichnet, daß man die Veretherung mit 2,3-Dihydropyran in Methylenchlorid in Anwesenheit von Pyridinium-p-Toluolsulfonsäure durchgeführt wird.

7. Zwischenverbindungen der allgemeinen Formel

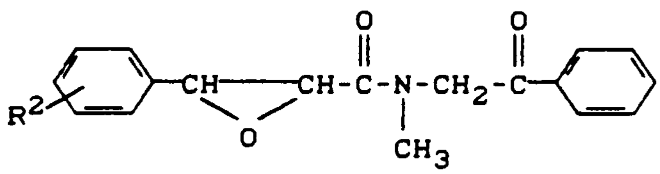

(II)

in welcher
$R^2$
- für Wasserstoff, Fluor, Chlor oder Brom steht.

8. Verfahren zur Herstellung der Zwischenverbindungen der Formel

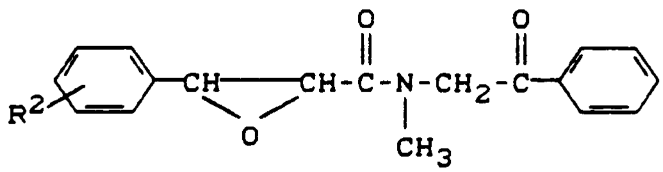

(II)

in welcher
$R^2$
- für Wasserstoff, Fluor, Chlor oder Brom steht,
dadurch gekennzeichnet, daß man
[A] Verbindungen der allgemeinen Formel,

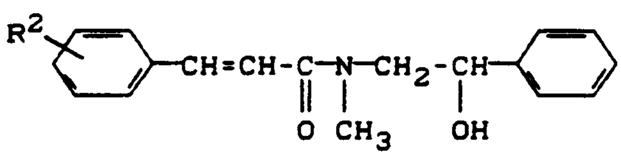

(VI)

in welcher
$R^2$ die oben angegebene Bedeutung hat,
zunächst nach üblicher Methode durch Oxidation der Hydroxygruppe in Verbindungen der allgemeinen Formel,

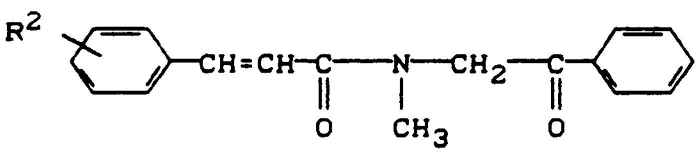

(VII)

in welcher
$R^2$ die oben angegebene Bedeutung hat,
überführt und in einem zweiten Schritt nach bekannter Methode eine Epoxidierung durchführt,
oder daß man

[B] Verbindungen der allgemeinen Formel,

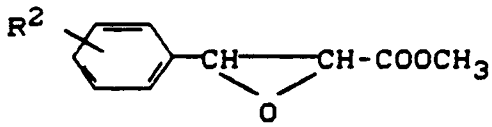

(VIII)

in welcher
$R^2$ die oben angegebene Bedeutung hat,
zunächst mit dem Amin der Formel

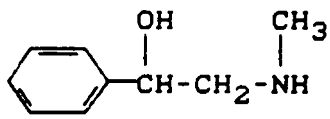

(IX)

nach üblicher Methode zu Verbindungen der allgemeinen Formel,

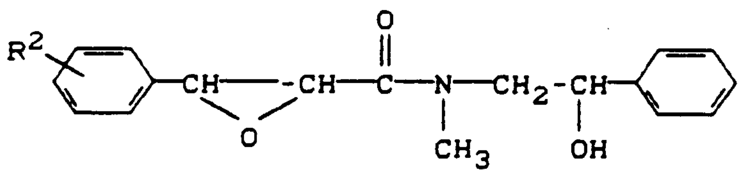

(X)

in welcher
$R^2$ die oben angegebene Bedeutung hat,
amidiert und in einem weiteren Schritt nach bekannter Methode die Hydroxygruppe oxidiert.

**9.** Verfahren nach Anspruch 8, dadurch gekennzeichnet, daß für die Oxidation der Hydroxygruppe organische oder anorganische Peroxoverbindungen wie beispielsweise Chlorperbenzoesäure oder Peroxoessigsäure, oder anorganische Oxide wie beispielsweise Chromoxide oder komplexe Chromoxide/Pyridin eingesetzt werden.

**10.** Verfahren nach Anspruch 8 und 9, dadurch gekennzeichnet, daß man die Oxidation der Hydroxygruppe in einem Temperaturbereich von +10 °C bis +70 °C durchführt.

## Claims

**1.** Process for the preparation of clausenamide, neoclausenamide and their derivatives of the general formula

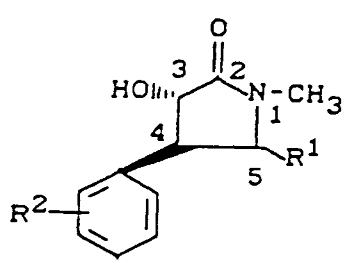

(I)

in which
$R^1$

- represents the group of the formula

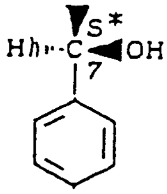

(clausenamide 3S*, 4R*, 5R*, 7S*), or

- represents the group of the formula

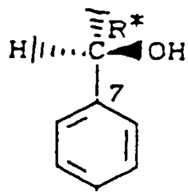

(neoclausenamide 3S*, 4R*, 5S*, 7R*), or

- represents the group of the formula

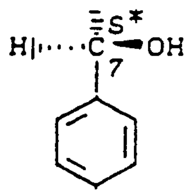

and

$R^2$

- represents hydrogen, fluorine, chlorine or bromine,

characterized in that compounds of the general formula

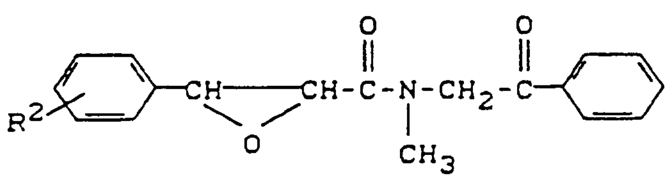

(II)

in which

$R^2$

- represents hydrogen, fluorine, chlorine or bromine,

are initially cyclized in inert organic solvents in the presence of a base, with the formation of the two isomers of the formulae

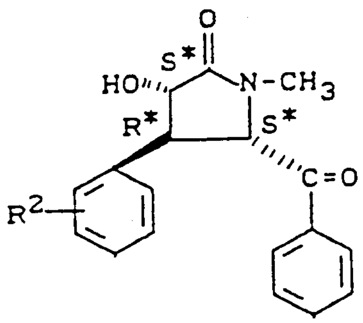

and

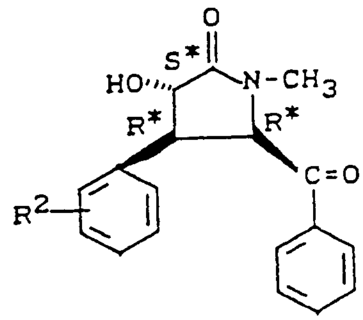

(III) (IV)

in which

$R^2$ has the abovementioned meaning,

and, in a further step, either

a) compounds of the general formula (IV) are reduced stereospecifically to give compounds of the general formula (I) in which $R^1$ represents the group of the formula

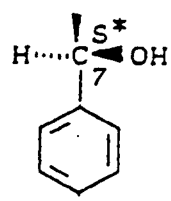

(clausenamide),

or in that

b), depending on the reaction conditions and the reducing agents chosen, compounds of the general formula (III) are reduced to give compounds of the general formula (I) in which $R^1$ represents the group of the formula

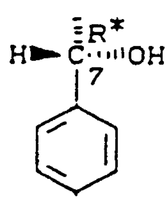

or

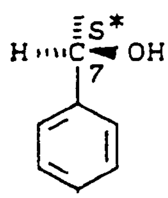

or in that

c) compounds of the general formula (III) are reacted with 2,3-dihydropyran to give compounds of the general formula

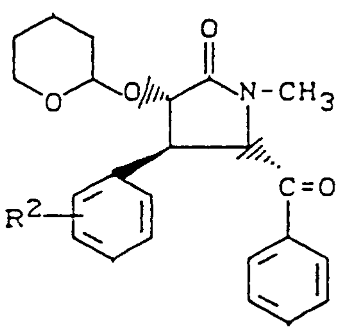

(V)

in which

$R^2$ has the abovementioned meaning,

and the product is then reduced stereoselectively to give compounds of the general formula (I) in which $R^1$ represents the group of the formula

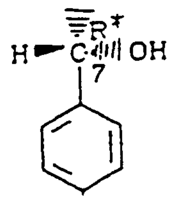

and, if appropriate, the isomers are separated by customary chromatographic methods.

**2.** Process according to Claim 1, characterized in that the solvents for the cyclization are inert organic solvents, such as ethers, for example tetrahydrofuran, diethyl ether or dioxane, or alcohols, such as, for example, methanol or ethanol, or halogenated hydrocarbons, such as, for example, methylene chloride or carbon tetrachloride, or their mixtures, if appropriate also with water.

**3.** Process according to Claims 1 and 2, characterized in that the bases employed in the cyclization are alkali metal alcoholates, alkali metal amides or alkali metal hydrides, such as, for example, sodium ethoxide, sodium methoxide, potassium butoxide, sodium butoxide or lithium butoxide, lithium hydroxide, sodium hydroxide or potassium hydroxide, sodium hydride, lithium diisopropylamide, butyllithium or ammonium hydroxides, such as, for example, tetramethylammonium hydroxide.

**4.** Process according to Claims 1 to 3, characterized in that the cyclization is carried out under the conditions of a 2-phase-transfer reaction.

**5.** Process according to Claims 1 to 4, characterized in that the reduction of the keto function to the hydroxyl function is carried out with the customary reducing agents, such as metallic hydrides, complex hydrides or with organic aluminium compounds.

**6.** Process according to Claims 1 to 5, characterized in that the etherification is carried out with 2,3-dihydropyran in methylene chloride in the presence of pyridinium p-toluenesulphonic acid.

**7.** Intermediates of the general formula

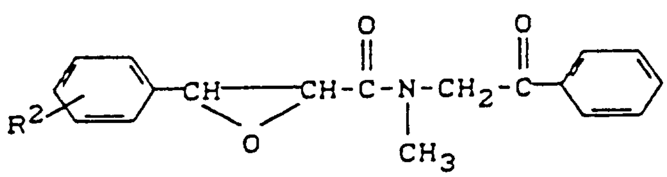

(II)

in which

$R^2$

- represents hydrogen, fluorine, chlorine or bromine.

**8.** Process for the preparation of the intermediates of the formula

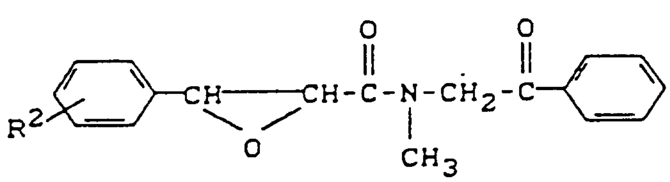

(II)

in which
$R^2$
- represents hydrogen, fluorine, chlorine or bromine,

characterized in that

[A] compounds of the general formula

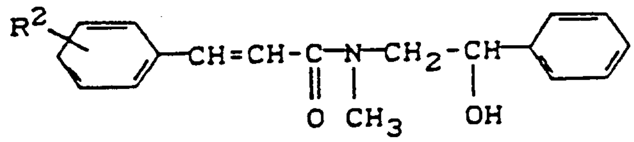

(VI)

in which
$R^2$ has the abovementioned meaning,

are initially converted into compounds of the general formula

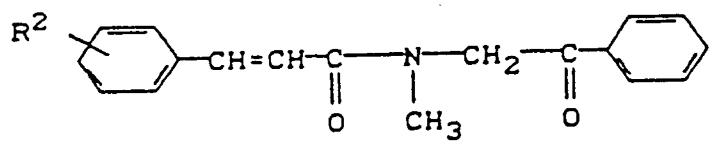

(VII)

in which
$R^2$ has the abovementioned meaning,

by oxidizing the hydroxyl group by a customary method, and, in a second step, an epoxidization is carried out by a customary method,

or in that

[B] compounds of the general formula

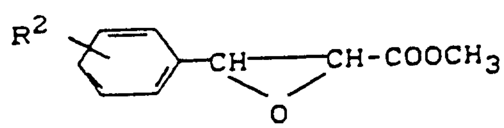

(VIII)

in which
$R^2$ has the abovementioned meaning,

are initially amidated with the amine of the formula

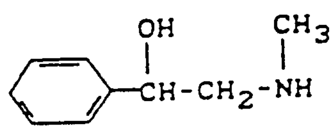

(IX)

in a customary manner to give compounds of the general formula

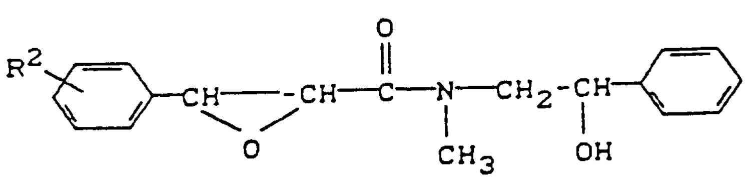

(X)

in which
$R^2$ has the abovementioned meaning,

and, in a further step, the hydroxyl group is oxidized in a known manner.

9. Process according to Claim 8, characterized in that organic or inorganic peroxo compounds such as, for example, chloroperbenzoic acid or peroxyacetic acid, or inorganic oxides, such as, for example, chromium oxides or complex chromium oxides/pyridine are employed for the oxidation of the hydroxyl group.

10. Process according to Claims 8 and 9, characterized in that the oxidation of the hydroxyl group is carried out in a temperature range of from +10°C to +70°C.

**Revendications**

1. Procédé de production de clausénamide, de néoclausénamide et de leurs dérivés de formule générale

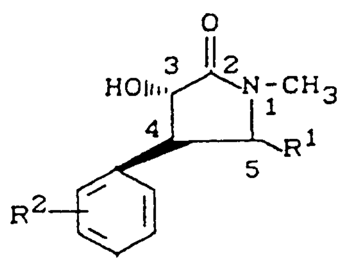

(I)

dans laquelle

R1

- représente le groupe de formule

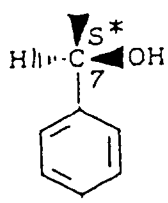

(clausénamide 3S*, 4R*, 5R*, 7S*)

ou

- le groupe de formule

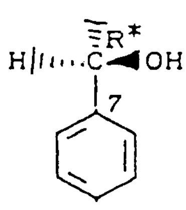

(néoclausénamide 3S*, 4R*, 5R*, 7S*),

ou bien

- le groupe de formule

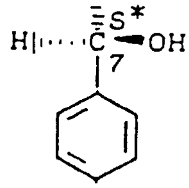

et

$R^2$ - représente de l'hydrogène, du fluor, du chlore ou du brome,

caractérisé en ce qu'on cyclise tout d'abord des composés de formule générale

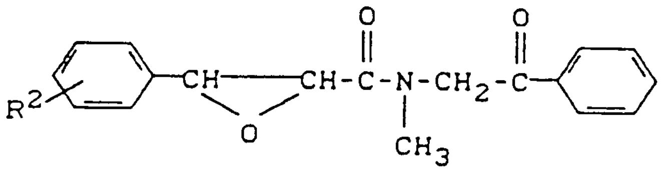

(II)

(II)

dans laquelle

$R^2$ - est de l'hydrogène, du fluor, du chlore ou du brome,

dans des solvants organiques inertes, en présence d'une base, avec formation des deux isomères de formules

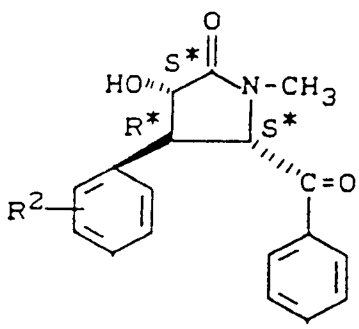

et

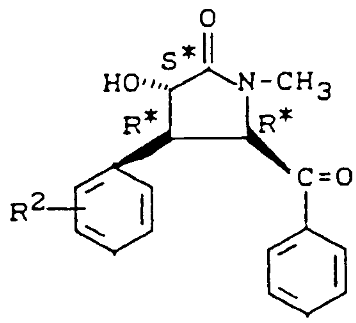

(III) (IV)

dans laquelle

$R^2$ a la définition indiquée ci-dessus,

et dans une autre étape,

a) on réduit des composés de formule générale (IV) stéréospécifiquement en composés de formule générale (I), dans laquelle $R^1$ représente le groupe de formule

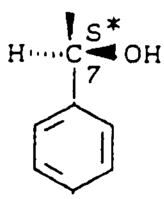

(clausénamide),

ou bien

b) on réduit des composés de formule générale (III), selon le choix des conditions réactionnelles et des agents réducteurs, en composés de formule générale (I) dans laquelle $R^1$ représente le groupe de formule

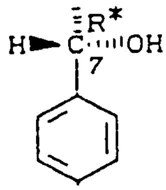

ou

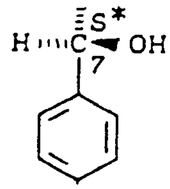

ou bien

c) on fait réagir des composés de formule générale (III) avec le 2,3-dihydropyranne pour produire des composés de formule générale

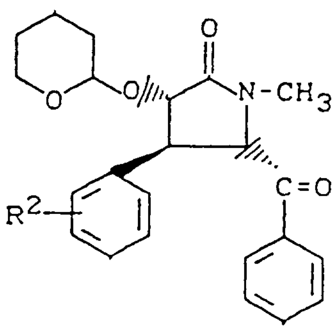

(V)

dans laquelle

$R^2$ a la définition indiquée ci-dessus,

puis on réduit stéréosélectivement ces composés en composés de formule générale (I) dans laquelle $R^1$ représente le groupe de formule

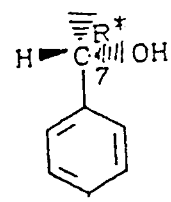

et le cas échéant, on sépare éventuellement les isomères par une méthode chromatographique classique.

**2.** Procédé suivant la revendication 1, caractérisé en ce qu'on utilise comme solvants pour la cyclisation, des solvants organiques inertes tels que des éthers, par exemple le tétrahydrofuranne, l'éther de diéthyle ou le dioxanne, ou des alcools tels que, par exemple, le méthanol ou l'éthanol, ou des hydrocarbures halogénés tels que, par exemple, le chlorure de méthylène ou le tétrachlorure de carbone, ou leurs mélanges, ainsi que l'eau le cas échéant.

**3.** Procédé suivant la revendication 1 ou 2, caractérisé en ce qu'on utilise comme bases pour la cyclisation des alcoolates alcalins, des amidures alcalins ou des hydrures alcalins tels que, par exemple, l'éthanolate de sodium, le méthanolate de sodium, le butanolate de potassium, sodium ou lithium, l'hydroxyde de lithium, sodium ou potassium, l'hydrure de sodium, le diisopropylamidure de lithium, le butyl-lithium ou des hydroxydes d'ammonium tels que, par exemple, l'hydroxyde de tétraméthylammonium.

**4.** Procédé suivant les revendications 1 à 3, caractérisé en ce qu'on conduit la cyclisation dans les conditions d'une réaction de transfert à deux phases.

**5.** Procédé suivant les revendications 1 à 4, caractérisé en ce qu'on conduit la réduction de la fonction céto en foncion hydroxy avec les agents réducteurs classiques tels que des hydrures métalliques, des

hydrures complexes, ou avec des composés organiques d'aluminium.

6. Procédé suivant les revendications 1 à 5, caractérisé en ce qu'on conduit l'éthérification avec le 2,3-dihydropyranne dans du chlorure de méthylène en présence d'acide pyridium-p-toluène-sulfonique.

7. Composés intermédiaires de formule générale

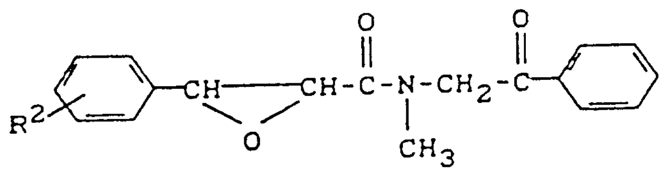
(II)

dans laquelle
$R^2$
- représente de l'hydrogène, du fluor, du chlore ou du brome.

8. Procédé de production de composés intermédiaires de formule

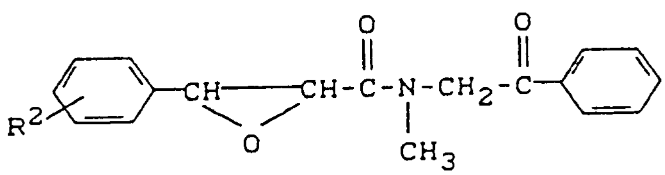
(II)

dans laquelle
$R^2$
- représente de l'hydrogène, du fluor, du chlore ou du brome,

caractérisé en ce que
[A] on transforme tout d'abord des composés de formule générale

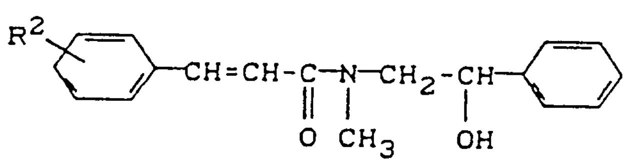
(VI)

dans laquelle
$R^2$ a la définition indiquée ci-dessus,

par des procédés classiques, par oxydation du groupe hydroxy, en composés de formule générale

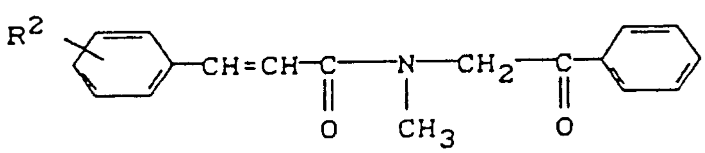
(VII)

dans laquelle
$R^2$ a la définition indiquée ci-dessus,

et on conduit dans une seconde étape une époxydation par un procédé connu,
ou bien

[B] on effectue tout d'abord une amidation de composés de formule générale

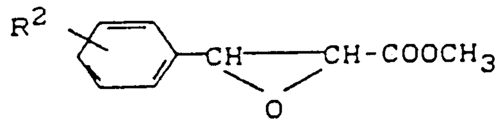

(VIII)

dans laquelle

$R^2$ a la définition indiquée ci-dessus,

avec l'amine de formule

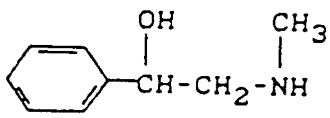

(IX)

par un procédé classique en composés de formule générale

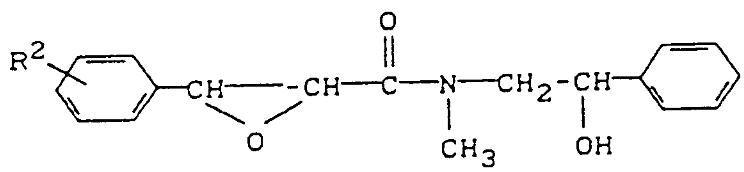

(X)

dans laquelle

$R^2$ a la définition indiquée ci-dessus,

et, dans une autre étape, on oxyde le groupe hydroxy par un procédé connu.

**9.** Procédé suivant la revendication 8, caractérisé en ce qu'on utilise pour l'oxydation du groupe hydroxy, des composés peroxo organiques ou inorganiques tels que, par exemple, l'acide chloroperbenzoïque ou l'acide peroxoacétique, ou des oxydes inorganiques tels que, par exemple, des oxydes de chrome ou des complexes oxydes de chrome/pyridine.

**10.** Procédé suivant les revendications 8 et 9, caractérisé en ce qu'on conduit l'oxydation du groupe hydroxy dans une plage de températures de +10 °C à +70 °C.